# EUROPEAN PATENT APPLICATION

(11) **EP 3 158 938 A1**
(43) Date of publication of application: **26.04.2017**
(21) Application number: 15810379.6
(22) Date of filing: 21.04.2015
(51) Int. Cl.: A61B 8/00

(54) **ULTRASONIC DIAGNOSTIC DEVICE**

(30) Priority: 17.06.2014 JP 2014124536
(71) Applicant: Hitachi, Ltd., Chiyoda-ku, Tokyo 100-8280 (JP)
(72) Inventor: YOSHIZAWA, Shingo, Mitaka-shi Tokyo 181-8622 (JP); HISATSU, Masanori, Mitaka-shi Tokyo 181-8622 (JP)
(74) Representative: Strehl Schübel-Hopf & Partner
(86) International application number: PCT/JP2015/062133
(87) International publication number: WO 2015/194253

(57) **Abstract**

Provided is an ultrasonic diagnostic device in which a transmission unit (12) outputs a transmission signal to a probe (10) such that an ultrasonic wave is transmitted a plurality of times while the position of a sound source is changed. A reception unit (14) obtains the received ultrasonic wave signal from the probe (10) for each of the plurality of times that the transmission signal is transmitted. A synthesis processing unit (20) synthesizes the received ultrasonic wave signals that are obtained over the plurality of times. A higher harmonic extraction unit (30) extracts a higher harmonic component from the synthesized received ultrasonic wave signal. An image forming unit (40) forms an ultrasonic wave image on the basis of the extracted higher harmonic component. A control unit (50) adjusts the time interval of the plurality of times that the ultrasonic wave is transmitted and controls the transmission voltage of the transmission signal.

## Description

### TECHNICAL FIELD

The present disclosure relates to an ultrasound diagnostic apparatus, and in particular to an apparatus which uses harmonics of ultrasound.

### BACKGROUND

There is known a harmonic imaging technique which uses harmonic components which are generated by a nonlinear effect of ultrasound which propagates within a living body. Ultrasound diagnostic apparatuses equipped with the function of the harmonic imaging are widely in use because the device is not easily affected by an artifact such as multiple echo because a propagation distance of the ultrasound is short and harmonic components are not generated in a large amount near a surface of the body, that contrast resolution can be improved because a side lobe is reduced because the harmonic component is generated proportional to a square of sound pressure, or the like.

In addition, as a technique related to transmission and reception of the ultrasound, synthetic transmit aperture is known (for example, refer to Patent Document 1). Synthetic transmit aperture has attracted much attention as an alternative technique of a method in which an ultrasound beam is formed and scanned by phasing addition process or the like.

### CITATION LIST

### PATENT LITERATURE

Patent Document 1: JP 2009-101165 A

### SUMMARY

### TECHNICAL PROBLEM

In view of the related art described above, the present inventors have repeated research and development for a technique related to harmonics of the ultrasound such as, for example, harmonic imaging, and in particular, has looked into combining a technique related to the harmonics of the ultrasound and a technique related to the synthetic transmit aperture.

The present disclosure has been made in the research and development, and an advantage thereof lies in provision of an improved technique combining a technique related to the harmonics of the ultrasound and a technique related to synthetic transmit aperture.

### SOLUTION TO PROBLEM

According to one aspect of the present disclosure, there is provided an ultrasound diagnostic apparatus comprising: a probe that transmits and receives ultrasound; a transmitter that outputs a transmission signal to the probe to transmit ultrasound a plurality of times while changing a position of a sound source; a receiver that obtains a reception signal of ultrasound from the probe for each time of the transmission over the plurality of times; a synthesis processor that synthesizes reception signals of ultrasound obtained over the plurality of times; a harmonic extractor that extracts a harmonic component from the synthesized reception signal of ultrasound; an image former that forms an ultrasound image based on the extracted harmonic component; and a controller that adjusts a time interval of the transmission over the plurality of times and controls a transmission voltage of the transmission signal.

In the above-described apparatus, the probe desirably includes a plurality of transducer elements each of which transmits and receives ultrasound. The position of the sound source may be, for example, on a transmission and reception surface of the probe (including a region nearby), or a position distanced away from the probe; for example, a transmission focus position, may be set as a virtual sound source. Moreover, the harmonic component may be extracted using a band-pass filter (high-pass filter) or by a known method such as pulse inversion or the like.

According to the above-described apparatus, when the transmission voltage is controlled, by adjusting the time interval of transmission, for example, an allowable range of the transmission voltage can be changed. With such a configuration, for example, it becomes possible to control the transmission voltage so that a desired transmission energy is not exceeded.

According to another aspect of the present disclosure, the controller controls the transmission voltage in such a manner that the transmission voltage is √m times a standard voltage which is set as a standard state, wherein m is a real number greater than 1. With such a configuration, for example, a signal value of a harmonic component can be set to be m times that of the standard state.

According to another aspect of the present disclosure, in controlling the transmission voltage to be √m times the standard voltage, the controller adjusts the time interval of transmission in such a manner that the time interval is m times a standard time which is set as a standard state. With such a configuration, for example, the signal value of the harmonic component can be set to be m times that of the standard state while maintaining a transmission energy of the standard state.

According to another aspect of the present disclosure, in adjusting the time interval of transmission over the plurality of times and controlling the transmission voltage of the transmission signal, the controller sets a number of transmissions according to the adjusted time interval of transmission. By setting the number of transmissions according to the adjusted time interval of transmission, it becomes possible, for example, to suppress a change in a frame rate due to the adjustment of the time interval of transmission.

According to another aspect of the present disclosure, the controller adjusts the time interval of transmission in such a manner that the time interval is m times a standard time which is set as a standard state, wherein m is a real number greater than 1, and sets the number of transmissions in such a manner that the number of transmissions is 1/m times a standard number which is set as a standard state. With such a configuration, for example, the frame rate of the standard state can be maintained.

According to another aspect of the present disclosure, the controller adjusts the time interval of transmission in such a manner that the time interval is m times a standard time which is set as a standard state, sets the number of transmissions in such a manner that the number of transmissions is 1/m times a standard number which is set as a standard state, and controls the transmission voltage in such a manner that the transmission voltage is √m times a standard voltage which is set as a standard state. With such a configuration, for example, a signal-to-noise ratio of the harmonic component can be set to be √m times that of the standard state while maintaining the transmission energy and the frame rate of the standard state.

### ADVANTAGEOUS EFFECTS OF INVENTION

According to various aspects of the present disclosure, an improved technique is provided by combining the technique related to the harmonics of the ultrasound and the technique related to the synthetic transmit aperture.

### BRIEF DESCRIPTION OF DRAWINGS

FIG. 1 is a diagram showing the overall structure of an ultrasound diagnostic apparatus according to an embodiment of the present disclosure.
FIG. 2 is a diagram for explaining a first specific configuration of synthetic transmit aperture.
FIG. 3 is a diagram for explaining a second specific example of synthetic transmit aperture.
FIG. 4 is a diagram showing a specific example control by a controller.

### DESCRIPTION OF EMBODIMENTS

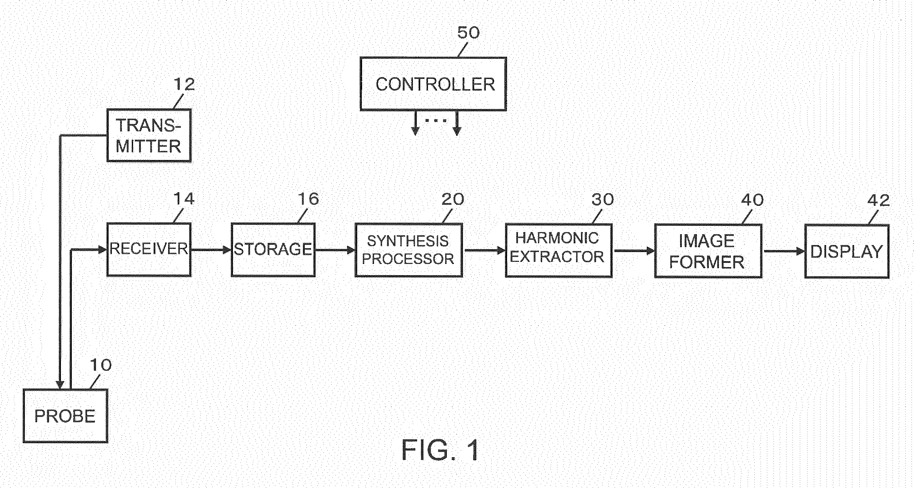

FIG. 1 is an overall structural diagram of an ultrasound diagnostic apparatus according to an embodiment of the present disclosure. A probe 10 is an ultrasound probe which transmits and receives ultrasound to and from a region including a diagnosis target. The probe 10 comprises a plurality of transducer elements which transmit and receive ultrasound, and is desirably, for example, a linear-type or sector-type ultrasound probe, but may alternatively be an ultrasound probe of another type.

A transmitter 12 outputs a transmission signal to the probe 10, to transmit ultrasound over a plurality of times while changing a position of a sound source. A receiver 14 obtains a reception signal of ultrasound from the probe 10 for each time of the transmission over the plurality of times. The reception signals of ultrasound obtained over the plurality of times are stored in a storage 16.

A synthesis processor 20 applies a synthesis process of the reception signals (reception signal data) stored in the storage 16; that is, the ultrasound reception signals obtained over the plurality of times. The transmitter 12, the receiver 14, and the synthesis processor 20 execute a transmission and reception process called synthetic transmit aperture, to obtain a synthesized ultrasound reception signal.

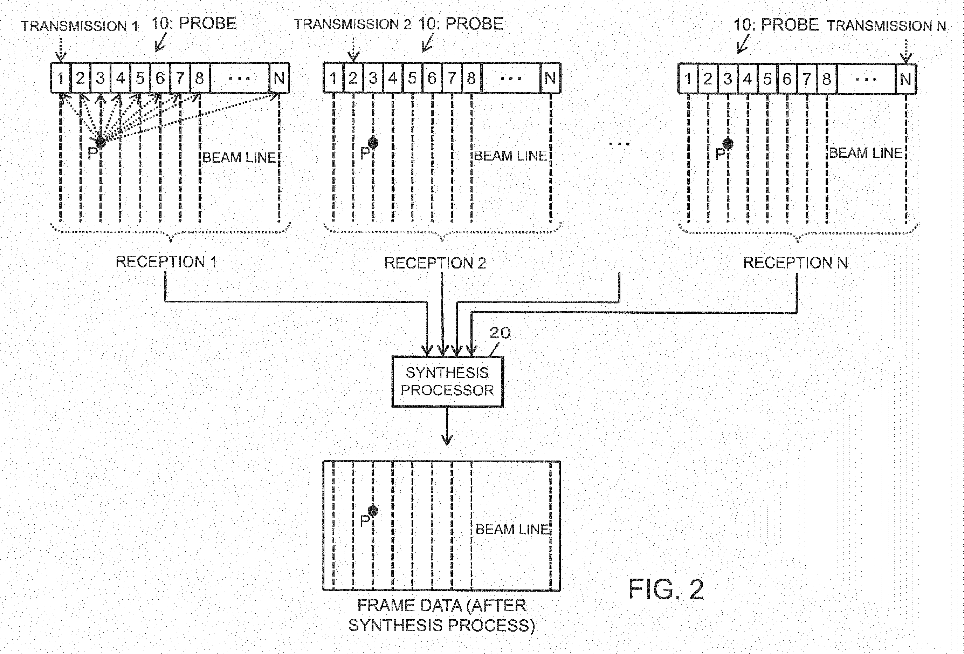

FIG. 2 is a diagram for explaining a first specific configuration of the synthetic transmit aperture. FIG. 2 shows a specific example configuration in which transmissions of N times from transmission 1 to transmission N (wherein N is a natural number) are executed while changing the position of the sound source, reception signals of N times from reception 1 to reception N are obtained, and a synthesis process of the N reception signals is executed to obtain frame data after the synthesis process. In FIG. 2, the probe 10 comprises N transducer elements, which are each shown with a rectangle and with an element number (1 ∼ N) shown within the rectangle.

In the first specific configuration shown in FIG. 2, in the transmission of each time, ultrasound is transmitted from one transducer element, and in the reception of each time, ultrasound is received by N transducer elements. For example, in the transmission of a first time, ultrasound is transmitted only from the transducer 1, and a reflection involved with the transmitted ultrasound is received by each of the transducer elements 1 ∼ N. In other words, the ultrasound reception signal is obtained at each of the transducer elements 1 ∼ N. In the transmission of a second time, the ultrasound is transmitted only from the transducer 2, and a reflection involved with the transmitted ultrasound is received by each of the transducer elements 1 ∼ N. Similarly, transmissions and receptions of third and later times are repeated. In the transmission of an Nth time, the ultrasound is transmitted only from the transducer element N, and the ultrasound reception signal is obtained by each of the transducer elements 1 ∼ N.

In addition, in the first specific configuration of FIG. 2, in the reception of each time, N reception beams corresponding to N beam lines shown by broken lines are formed. Specifically, based on the reception signals received by the transducer elements 1 ∼ N, each reception beam is formed with each of a plurality of sample points on each beam line as a reception focus, and a reception signal corresponding to each beam line (reception beam signal) is obtained. FIG. 2 shows a sample point P as a representative of the plurality of sample points on each beam line.

The synthesis processor 20 applies a synthesis process on the reception signals over the plurality of times from the reception 1 to reception N, to obtain frame data formed from the synthesized reception signals. In the first specific configuration of FIG. 2, the synthesis processor 20 applies an addition process on the reception signals of each of the sample points included in the reception signals (reception beam signals) from the reception 1 to the reception N, to obtain a reception signal after the synthesis process related to the sample point. For example, N reception signals at a sample point P included in the reception signals from the reception 1 to the reception N are added, to obtain the reception signal after the synthesis process at the sample point P.

With the first specific configuration of FIG. 2, it is possible to obtain the synthesis-processed frame data formed from reception signals at a plurality of sample points while realizing both transmission focus and reception focus at each sample point.

Alternatively, in place of forming the reception beam at the reception of each time, for example, it is also possible to obtain a reception signal from each of the transducer elements 1 ∼ N at the reception of each time, and the synthesis process may be applied to the reception signals over N times from reception 1 to reception N, to obtain the synthesis-processed frame data.

Alternatively, transmissions of M times (wherein M is a natural number smaller than N) may be executed dispersively by M transducer elements with several transducer elements therebetween, ultrasound may be received by N transducer elements at the reception of each time, and the reception signals obtained over M times may be synthesized to obtain the synthesis-processed frame data. In executing the transmission of M times, for example, the positions of the sound sources (transducer elements transmitting the ultrasound) are desirably at a constant interval, but the interval of the sound sources is not limited to a constant interval.

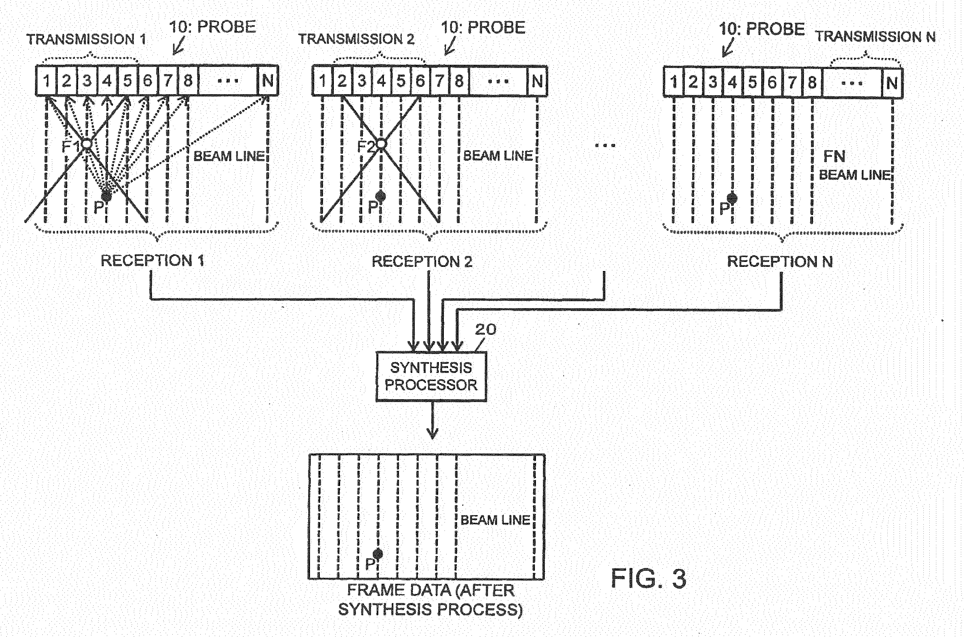

FIG. 3 is a diagram for explaining a second specific configuration of the synthetic transmit aperture. In the second specific configuration of FIG. 3, a virtual sound source is realized by transmission focus, transmissions of N times from transmission 1 to transmission N (wherein N is a natural number) are executed while changing the position of the virtual sound source, reception signals of N times from reception 1 to reception N are obtained, and the reception signals of N times are synthesized to obtain frame data after the synthesis process. The probe 10 shown in FIG. 3 also includes N transducer elements, each of which is shown with a rectangle and with an element number (1 ∼ N) shown within the rectangle.

In the second specific configuration of FIG. 3, ultrasound is transmitted from several transducer elements in order to realize the transmission focus at the transmission of each time, and the ultrasound is received by N transducer elements at the reception of each time. For example, in the transmission of a first time, ultrasound is transmitted from transducer elements 1 ∼ 5 with a transmission focus F1 as the focal point, and a reflection involved with the transmitted ultrasound is received by each of the transducer elements 1 ∼ N. In other words, a reception signal of the ultrasound is obtained at each of the transducer elements 1 ∼ N. In the transmission of a second time, ultrasound is transmitted from the transducer elements 2 ∼ 6 with a transmission focus F2 as the focal point, and a reflection involved with the transmitted ultrasound is received at each of the transducer elements 1 ∼ N. similarly, the transmission and reception of third and later times are repeated, and, at the transmission of an Nth time, ultrasound is transmitted from the transducer element N and nearby transducer elements with a transmission focus FN as the focal point, and a reception signal of the ultrasound is obtained at each of the transducer elements 1 ∼ N.

In addition, in the second specific configuration of FIG. 3, in the reception of each time, N reception beams corresponding to N beam lines shown with broken lines are formed. Specifically, based on the reception signals received by the transducer elements 1 ∼ N, each reception beam is formed with each of a plurality of sample points on each beam line as a reception focus, and a reception signal (reception beam signal) corresponding to each beam line is obtained. FIG. 3 shows a sample point P as a representative of the plurality of sample points on each beam line.

The synthesis processor 20 applies the synthesis process on the reception signals over the plurality of times from the reception 1 to the reception N, to obtain frame data formed from the synthesized reception signal. Similar to the first specific configuration of FIG. 2, in the second specific configuration of FIG. 3 also, the synthesis processor 20 applies an addition process on the reception signals of each sample point included in the reception signals (reception beam signals) from the reception 1 to the reception N, to obtain the synthesized reception signal related to the sample point. For example, N reception signals at the sample point P included in the reception signals from the reception 1 to the reception N are added, to obtain the synthesis-processed reception signal at the sample point P.

Alternatively, in place of forming the reception beam at the reception of each time, for example, a reception signal may be obtained from each of the transducer elements 1 ∼ N at the reception of each time, and the reception signals over N times from the reception 1 to the reception N may be synthesized to obtain the synthesis-processed frame data.

Alternatively, the interval of the virtual sound source (transmission foci F1, F2, ...) may be widened, and, for example, transmission of M times (wherein M is a natural number smaller than N) may be executed with each of M transmission foci F1 ∼ FM as the focal point, ultrasound may be received by N transducer elements at the reception of each time, and the reception signals obtained over M times may be synthesized, to obtain the synthesis-processed frame data. In executing the transmission of M times, for example, the positions of the virtual sound sources (transmission foci F1 ∼ FM) are desirably at a constant interval, but the interval of the sound sources is not limited to the constant interval.

Referring back to FIG. 1, a harmonic extractor 30 extracts a harmonic component included in the synthesized reception signal obtained from the synthesis processor 20. The harmonic extractor 30 obtains synthesized frame data (refer to FIGs. 2 and 3) from the synthesis processor 20, and extracts a harmonic component (for example, a second-order harmonic component) from the synthesized reception signal included in the frame data, for example, the synthesized reception signal corresponding to each beam line. The harmonic component may be extracted, for example, using a band-pass filter (high-pass filter) or by a known method such as pulse inversion or the like.

An image former 40 forms an ultrasound image (image data) based on the harmonic component extracted by the harmonic extractor 30. The image former 40 forms the ultrasound image of, for example, the harmonic imaging. The ultrasound image formed by the image former 40 is displayed on a display 42.

A controller 50 controls the overall ultrasound diagnostic apparatus shown in FIG. 1. The ultrasound diagnostic apparatus of FIG. 1 desirably includes an operation device such as, for example, a mouse, a keyboard, a trackball, a touch panel, a joystick, or the like. In the overall control by the controller, commands received from the user through the operation device or the like are also reflected.

Of the structures shown in FIG. 1 (units with reference numerals attached), each of the transmitter 12, the receiver 14, the synthesis processor 20, the harmonic extractor 30, and the image former 40 may be realized using hardware such as, for example, an electronic or electric circuit, a processor, or the like, and a device such as a memory may be used as necessary in realizing these units. Alternatively, the functions corresponding to these units may be realized by a cooperation of hardware such as a CPU, a processor, and a memory, and software (program) defining operations of the CPU and the processor.

Desirable specific examples of the storage 16 include a semiconductor memory, a hard disk drive, or the like, and desirable specific examples of the display 42 include a liquid crystal display or the like. The controller 50 can be realized, for example, by a cooperation of hardware such as a CPU, a processor, and a memory, and software (program) defining operations of the CPU and the processor.

In the ultrasound diagnostic apparatus of FIG. 1, when the controller 50 uses the synthetic transmit aperture in the harmonic imaging, the controller 50 adjusts a time interval of transmission over a plurality of times, to control a transmission voltage of the transmission signal. Further, in adjusting the time interval of the transmission over the plurality of times and controlling the transmission voltage of the transmission signal, the controller 50 adjusts the number of transmissions according to the adjusted time interval of transmission.

The transmission voltage is a maximum voltage of the transmission signal which is output from the transmitter 12 to each transducer of the probe 10 (for example, a maximum voltage within a transmission pulse waveform), the time interval of transmission is a time between transmissions in the transmission over a plurality of times in the synthetic transmit aperture (that is, a pulse repetition period: PRT), and the number of transmissions is the number of transmissions in the synthetic transmit aperture.

In the ultrasound diagnostic apparatus, from the viewpoint of safety with respect to the subject, various restrictions (for example, Ispta, MI, TI, probe surface temperature increase, or the like) are applied on design. Of various restrictions, in particular, the probe surface temperature increase is in many cases the strictest condition. The probe surface temperature increase is proportional to an electric power energy introduced to the probe 10. Specifically, the probe surface temperature increase is proportional to a product of introduced electric power and 1/PRT, and thus, is proportional to a product of a square of the transmission voltage and 1/PRT.

In general, PRT is determined according to a roundtrip time of the ultrasound to and from a depth to be diagnosed. Thus, a PRT which is set as a standard in the apparatus with the depth to be diagnosed as a reference; that is, a PRT (time interval of transmission) which is set as a standard state, is referred to as a standard PRT. In addition, a maximum transmission voltage according to the restriction of the probe surface temperature increase (a value which does not exceed the limit) at the standard PRT is referred to as a standard transmission voltage V.

In the ultrasound diagnostic apparatus of FIG. 1, when the controller 50 uses the synthetic transmit aperture in the harmonic imaging, the controller 50 sets a longer PRT (larger PRT) than the standard PRT. For example, the PRT (time interval of transmission) in the synthetic transmit aperture is set to be m times (wherein m is a real number greater than 1) the standard PRT.

By setting the PRT to be m times the standard PRT, the maximum transmission voltage according to the restriction of the probe surface temperature increase can be increased to √m times the standard transmission voltage V This is because the probe surface temperature increase is proportional to the electric power energy introduced to the probe 10, and thus is proportional to a product of a square of the transmission voltage and 1/PRT.

Further, the controller 50 adjusts the number of transmissions according to the adjusted PRT (time interval of transmission). That is, the standard number of transmissions which is set as the standard state is adjusted. The controller 50 sets, for example, the number of transmissions to 1/m times the standard number of transmissions when the controller 50 sets the PRT to be m times the standard PRT. With this process, the frame rate at the standard state can be maintained.

When the transmission voltage is set at √m times the standard transmission voltage V, the harmonic signal (harmonic component extracted from the reception signal) becomes m times that in the standard transmission voltage V This is because the harmonic signal (for example, the second-order harmonic component) is proportional to the square of the transmission voltage. Therefore, when the transmission voltage becomes √m times the standard transmission voltage V, because the electrical noise is unchanged, an SNR (signal-to-noise ratio) of the harmonic signal becomes m times that in the case of the standard transmission voltage V.

On the other hand, in the synthetic transmit aperture, when the number of transmission is set 1/m times the standard number of transmissions, the SNR becomes 1/√m times that in the case of the standard number of transmissions.

With the combined improvement (m times) of the SNR due to the adjustment of the transmission voltage and the change (1/√m times) of the SNR due to the adjustment of the number of transmissions, the SNR becomes √m times that of the standard state. In other words, in the harmonic imaging by synthetic transmit aperture, the SNR can be improved while complying with the restrictions on the probe surface temperature increase. Further, as already described, the frame rate at the standard state can also be maintained.

In general, as the number of transmissions is reduced in the synthetic transmit aperture, the transmission and reception characteristics of the ultrasound are degraded, and artifacts such as a side lobe and a grating lobe tend to appear more often. In the harmonic imaging, because the number of these artifacts is originally small, the reduction in the number of transmissions in the synthetic transmit aperture can be permitted to a certain degree. However, when the number m is set too large; that is, when the number of transmissions is reduced excessively, the influence of the artifacts or the like would be increased to a degree that the artifacts cannot be ignored. Thus, it is desirable to suitably set the number m according to a balance between the improvement of the SNR of the image and the artifacts or the like appearing in the image.

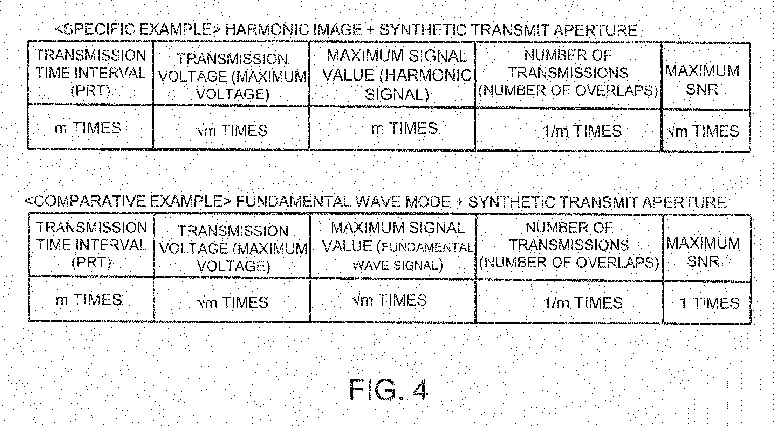

FIG. 4 is a diagram showing a specific example control by the controller 50. FIG. 4 shows a specific example control related to the transmission time interval (PRT) or the like when the synthetic transmit aperture is used in the harmonic imaging.

As already described, the controller 50 sets the transmission time interval (PRT) to be m times the standard PRT, the maximum transmission voltage to be √m times the standard transmission voltage, and the number of transmissions to be 1/m times the standard number of transmissions. With such a configuration, the harmonic signal becomes m times that in the case of the standard transmission voltage V, and the SNR becomes √m times that in the case of the standard state.

FIG. 4 also shows a comparative example of a case where the synthetic transmit aperture is used in the fundamental wave mode, for comparison with the harmonic imaging. In the fundamental wave mode, the fundamental wave component (fundamental wave signal) included in the reception signal is used. When the transmission voltage is set to √m times the standard transmission voltage V, the fundamental wave component becomes √m times that in the case of the standard transmission voltage V. Therefore, in the fundamental wave mode, when the transmission voltage is set to be √m times the standard transmission voltage V, the SNR (signal-to-noise ratio) becomes √m times that in the case of the standard transmission voltage V On the other hand, when the number of transmissions is set to be 1/m times the standard number of transmissions in the synthetic transmit aperture, the SNR becomes 1/√m times that in the case of the standard number of transmissions. In the fundamental wave mode, because of the improvement (√m times) of the SNR due to the adjustment of the transmission voltage and the change (1/√m times) of the SNR due to the adjustment of the number of transmissions, the total SNR becomes 1 times, and the SNR is not improved.

An embodiment of the present disclosure has been described. The above-described embodiment is merely exemplary from all aspects, and does not limit the scope of the present disclosure. The present disclosure includes various modifications within the scope and spirit thereof.

### REFERENCE SIGNS LIST

10 PROBE; 12 TRANSMITTER; 14 RECEIVER; 16 STORAGE; 20 SYNTHESIS PROCESSOR; 30 HARMONIC EXTRACTOR; 40 IMAGE FORMER; 42 DISPLAY; 50 CONTROLLER.

## Claims

1. An ultrasound diagnostic apparatus comprising:
a probe that transmits and receives ultrasound;
a transmitter that outputs a transmission signal to the probe to transmit ultrasound over a plurality of times while changing a position of a sound source;
a receiver that obtains a reception signal of ultrasound from the probe for each time of the transmission over the plurality of times;
a synthesis processor that synthesizes reception signals of ultrasound obtained over the plurality of times;
a harmonic extractor that extracts a harmonic component from the synthesized reception signal of ultrasound;
an image former that forms an ultrasound image based on the extracted harmonic component; and
a controller that adjusts a time interval of the transmission over the plurality of times and controls a transmission voltage of the transmission signal.

2. The ultrasound diagnostic apparatus according to Claim 1, wherein
the controller controls the transmission voltage in such a manner that the transmission voltage is √m times a standard voltage which is set as a standard state, wherein m is a real number greater than 1.

3. The ultrasound diagnostic apparatus according to Claim 2, wherein
in controlling the transmission voltage to be √m times the standard voltage, the controller adjusts the time interval of transmission in such a manner that the time interval is m times a standard time which is set as a standard state.

4. The ultrasound diagnostic apparatus according to Claim 1, wherein
in adjusting the time interval of transmission over the plurality of times and controlling the transmission voltage of the transmission signal, the controller sets a number of transmissions according to the adjusted time interval of transmission.

5. The ultrasound diagnostic apparatus according to Claim 4, wherein
the controller adjusts the time interval of transmission in such a manner that the time interval is m times a standard time which is set as a standard state, wherein m is a real number greater than 1, and sets the number of transmissions in such a manner that the number of transmissions is 1/m times a standard number which is set as a standard state.

6. The ultrasound diagnostic apparatus according to Claim 5, wherein
the controller adjusts the time interval of transmission in such a manner that the time interval is m times a standard time which is set as a standard state, sets the number of transmissions in such a manner that the number of transmissions is 1/m times a standard number which is set as a standard state, and controls the transmission voltage in such a manner that the transmission voltage is √m times a standard voltage which is set as a standard state.

7. The ultrasound diagnostic apparatus according to Claim 3, wherein
in adjusting the time interval of transmission over the plurality of times and controlling the transmission voltage of the transmission signal, the controller sets a number of transmissions according to the adjusted time interval of transmission.

8. The ultrasound diagnostic apparatus according to Claim 7, wherein
the controller adjusts the time interval of transmission in such a manner that the time interval is m times a standard time which is set as a standard state, wherein m is a real number greater than 1, and sets the number of transmissions in such a manner that the number of transmissions is 1/m times a standard number which is set as a standard state.

9. The ultrasound diagnostic apparatus according to Claim 8, wherein
the controller adjusts the time interval of transmission in such a manner that the time interval is m times a standard time which is set as a standard state, sets the number of transmissions in such a manner that the number of transmissions is 1/m times a standard number which is set as a standard state, and controls the transmission voltage in such a manner that the transmission voltage is √m times a standard voltage which is set as a standard state.

10. The ultrasound diagnostic apparatus according to Claim 1, wherein
the probe comprises a plurality of transducer elements,
ultrasound is transmitted from one transducer element at transmission of each time, and
the transmitter changes the position of the sound source by changing a transducer element to transmit the ultrasound in the transmission over the plurality of times.

11. The ultrasound diagnostic apparatus according to Claim 1, wherein
the probe comprises a plurality of transducer elements,
ultrasound is transmitted from several transducer elements to realize transmission focus at transmission of each time, and
the transmitter changes the position of the sound source by changing a position of the transmission focus in the transmission over the plurality of times.

12. The ultrasound diagnostic apparatus according to Claim 1, wherein
the probe comprises a plurality of transducer elements, and
the receiver forms reception beams corresponding to a plurality of beam lines based on receptions received by the plurality of transducer elements of the probe, and obtains a reception signal corresponding to each beam line, for each time of the transmission over the plurality of times.
